# EUROPEAN PATENT SPECIFICATION

(11) **EP 1 640 716 B1**
(45) Date of publication and mention of the grant of the patent: **02.03.2011**
(21) Application number: 04718722.4
(22) Date of filing: 09.03.2004
(51) Int. Cl.: G01N 33/48, A61K 35/16, C12N 5/00

(54) **BLOOD CELL SEPARATION MEMBRANE AND BLOOD RETENTION TOOL INCLUDING THE SAME**
BLUTZELLENTRENNUNGSMEMBRAN UND BLUTRÜCKHALTUNGSWERKZEUG DIES BEINHALTEND
MEMBRANE DE SEPARATION DE CELLULES SANGUINES ET OUTIL DE RETENTION DE SANG COMPRENANT CETTE MEMBRANE

(30) Priority: 10.03.2003 JP 2003063847
(43) Date of publication of application: 29.03.2006
(73) Proprietor: ARKRAY INC., Kyoto-shi, Kyoto 601-8045 (JP)
(72) Inventor: TANAKA, Yoshiyuki,, Kyoto-shi, Kyoto 601-8045 (JP); SHIMADA, Kentarou,, Kyoto-shi, Kyoto 601-8045 (JP); OKAMOTO, Masashi,, Kyoto-shi, Kyoto 601-8045 (JP); NAKAMURA, Tsutomu, Kyoto-shi, Kyoto 601-8045 (JP)
(74) Representative: Goddard, Christopher Robert
(86) International application number: PCT/JP2004/003017
(87) International publication number: WO 2004/083852

(56) References cited:
- WO-A-99/25463
- JP-A- 5 209 877
- JP-A- 9 196 908
- JP-A- 2001 124 765
- JP-A- 2001 124 766
- JP-A- 2001 188 066
- US-A- 3 552 928
- MORIMOTO Y. ET AL.: 'Protective effects of neutral amino acids against amphipathic drug-induced hemolysis' BIOL. PHARM. BULL. vol. 18, no. 11, 1995, pages 1535 - 1538, XP002982709
- MORIMOTO Y ET AL.: 'Protective effects of some neutral amino acids against hypotonic hemolysis' BIOL. PHARM. BULL. vol. 18, no. 10, 1995, pages 1417 - 1422, XP002982710
- MORIMOTO Y. ET AL.: 'Effects of neutral amino acids on membrane affinity of chloropromazine' JOURNAL OF PHARMACEUTICAL SCIENCE AND TECHNOLOGY vol. 49, no. 3, 1989, JAPAN, pages 215 - 220, XP002982711
- NAKAMORI K. ET AL.: 'Effectiveness of taurine in protecting biomembrane against oxidant' CHEM. PHARM. BULL. vol. 38, no. 11, 1990, pages 3116 - 3119, XP002982712
- ARAKAWA K. ET AL.: 'Membrane stabilizing action of NCO-650 and its congeners' JPN. J. PHARMACOL. vol. 36, no. 3, 1984, pages 311 - 318, XP002982713
- ELFERNIK J.G.: 'Crystal-induced membrane damage: hydroxypatite crystal-induced hemolysis of erythrocytes' BIOCHEM. MED. METAB. BIOL. vol. 36, no. 1, 1986, pages 25 - 35, XP002982714
- LOGUE G.L.: 'Effect of heparin on complement activation and lysis of paroxysmal nocturnal hemoglobinuria (PNH) red cells' BLOOD vol. 50, no. 2, 1977, pages 239 - 247, XP002982715
- PATEL H.M. ET AL.: 'Lytic effect of heparin on liposomes: possible mechanism of lysis of red blood cells by heparin' BIOSCI. REP. vol. 3, no. 1, 1983, pages 39 - 46, XP002982716
- BODEMANN H.H.ET AL.: 'Transfer of tris buffer and effects on K+ loss in human red blood cells and reconstituted ghosts' BIOCHIM. BIOPHYS. ACTA vol. 772, no. 1, 1984, pages 77 - 83, XP002982717
- RAVAL P.J. ET AL.: 'Relationship of hemolysis buffer structure, pH and ionic strength to spontaneous contour smoothing of isolated erythrocyte membranes' BIOCHIM. BIOPHYS. ACTA vol. 983, no. 2, 1989, pages 230 - 240, XP002982718
- NODA TANAKA ET AL.: 'Zenketsu ni yoru seikagaku kensa no tame no kensa device' NIHON SOGO KENSHIN IGAKUKAI TAIKAI PROGRAM vol. 29, 25 January 2001 - 26 January 2001, page 251, 174, XP002982719
- ANONYMOUS: "Catalogue, Appendix C, "Basic Filtration Concepts", section "Pore Size"" 2009, PALL SPECIALTY MATERIALS , PORT WASHINGTON, NY, USA

## Description

### Technical Field

The present invention relates to a blood cell separation membrane for separating blood into blood cells and serum or plasma and to a blood retention tool used for retaining or testing blood.

### Background Art

In a blood test, a sheet-like blood testing tool (also referred to as a "test piece") to be used per specimen is used for various purposes. Examples of this testing tool include those that retain blood, from which blood to be tested is extracted; and those that are pre-impregnated with a reagent or the like. In the latter testing tools, the measurement can be carried out by, for example, dropping blood on the testing tool so that the blood reacts with the reagent and measuring the reaction by an optical or electrochemical method.

Such a blood testing tool has been used for various purposes in general clinical tests or the like. In addition, it actually is used in remote clinical testing systems, for example. In such a remote clinical testing system, a patient collects blood by himself at home, and the blood testing tool is impregnated with the blood. This then is dried, and the blood testing tool is mailed to a test institute such as a hospital for testing. The patient who mailed the blood then can be informed of the test result by mail or by visiting the hospital.

When the test item is a component of serum or plasma such as blood glucose or the like, blood needs to be separated into blood cells and serum or plasma in the blood testing tool. Thus, a conventional blood testing tool generally is provided with a blood cell separator such as a glass filter.

For example, WO 99/25463 relates to an apparatus and method for treating whole blood, US 3552928 relates to a whole blood separation system means and test system. EP 535485 concerns an improved device and method for separating and assaying whole blood, which separates the cellular components of whole blood from plasma or serum and assays the plasma or serum for a predetermined soluble constituent.

According to such a blood testing tool, it is possible to prepare a serum/plasma sample by, for example, separating blood into blood cells and serum/plasma by the blood cell separator and allowing the serum/plasma that has passed through the blood cell separator to develop in a development portion by capillary action. Other than this blood testing tool, a blood testing tool using an asymmetric porous membrane with pores whose sizes vary in the thickness direction has been developed recently (e.g., JP 11 (1999)-505327 A and JP 2001 188066 A). When blood is supplied to the asymmetric porous membrane from the side having larger pores, the blood penetrates in the thickness direction, during which blood cells are separated from the blood. Thus, plasma/serum comes out of the other side, which then can be collected. The blood testing tool having such an asymmetric porous membrane is advantageous in that the clogging of blood cells can be prevented.

However, in such conventional blood testing tools, the blood cell separation might involve hemolysis of the blood cells, so that the serum or the like might contain components of the blood cells. Morimoto et al. have written about the effects of neutral amino acids against haemolysis in Biol.Pharm.Bull. Vol. 18, No. 11 (1995) pp. 1535-8 and No. 10 (1995) pp. 1417-22, and in the Journal of Pharmaceutical Science and Technology, Vol. 49, No. 3 (1989), Japan, pp. 215-20. Also see discussion of membrane protection and stabilisation, and the effects of haemolysis and lysis on membranes by Nakamori et al. in Chem. Pharm. Bull., Vol. 38, No. 11 (1990), pp. 3116-9; by Arakawa et al. in J. Pharmacol., Vol. 36, No. 3 (1984), pp. 311-8; by Elefernik in Biochme. Med. Metab. Biol., Vol. 36, No. 1 (1986), pp. 25-35; by Logue in Blood, Vol. 50, Neo. 2 (1977), pp. 239-47; by Patel et al. in Biosci. Rep., Vol. 3, No. 1 (1983), pp. 39-46; by Bodemann et al. in Biochim. Biophys. Acta, Vol. 77.2, No. 1 (1984), pp. 77-83; by Raval et al. in Biochim. Biophys. Acta, Vol. 983, No. 2 (1989), pp. 230-40; and by Tanaka et al. in Nihon Sogo Kenshin Igakukai Taikai Program, Vol. 29, 25 January 2001-26 January 2001, pages 251 and 174.

On this account, adding an additive for preventing hemolysis to blood beforehand has been required (e.g., JP 9(1997)-196908 A, JP 2001 124766 A and JP 2001 124765 A). However, this poses a problem in that, although the hemolysis is prevented, it becomes difficult to measure serum/plasma components accurately because the hematocrit (Ht) of the collected serum/plasma is decreased drastically by the influence of the additive.

### Disclosure of Invention

With the foregoing in mind, it is an object of the present invention to provide a blood cell separation membrane that can separate blood into blood cells and serum/plasma while preventing hemolysis of the blood cells accompanying the blood cell separation, and also to provide a blood retention tool using such a blood cell separation membrane.

In order to achieve the above object, a blood cell separation membrane (also referred to as a "blood separation membrane") according to the present invention includes a porous membrane for separating blood into blood cells and serum or plasma. The blood cell separation membrane is characterized in that the porous membrane contains at least one hemolysis inhibitor selected from the group consisting of valine, leucine, silk extracts ε-aminohexanoic acid and tranexamic acid,

As described above, the blood cell separation membrane of the present invention can prevent the hemolysis of the blood cells by containing at least one of the above-described types of hemolysis inhibitors. Thus, a serum or plasma sample that has been separated from the blood by passing through the blood cell separation membrane is free from components of the blood cells. As a result, components of the serum and the like can be measured with excellent accuracy. Moreover, the chances that hemoglobin pigment might be contained in the sample due to the hemolysis also can be eliminated, so that it becomes possible to carry out the measurement with respect to the sample directly by an optical method, visual observation, or the like.

Furthermore, by preparing a serum/plasma sample using the blood cell separation membrane of the present invention, it becomes possible to measure various components with high accuracy. The inventors of the present invention conducted keen studies as to the decrease in hematocrit (Ht) and the deterioration of the measurement accuracy with regard to various test items in the case of using the above-described conventional blood cell separation membranes. As a result, the inventors found that the fact that the conventionally used additive for preventing hemolysis is glycine and it generally is contained at a high concentration of about 100 mg to 200 mg per unit volume (cm³) of a blood cell separation membrane is responsible for the above-described influences on the measurement. The inventors also found that, with regard to a test item such as GGT or the like in particular, the deterioration of the measurement accuracy occurs not only because serum or plasma is diluted with glycine but also because glycine denatures components of serum or plasma. In the case where the above-described hemolysis inhibitor is contained instead of glycine as in the blood cell separation membrane of the present invention, the concentration thereof need not be as high as that of glycine, for example. Based on this finding, the inventors found that the use of the above-described hemolysis inhibitor can reduce the influence on a Ht and allows various test items, including GGT, for example, to be measured with high accuracy. As described above, the blood cell separation membrane of the present invention can separate blood into blood cells and serum/plasma while preventing hemolysis of the blood cells accompanying the blood cell separation and thus can provide a serum sample or a plasma sample causing little influence on various analyses.

Next, a blood retention tool according to the present invention includes: a blood cell separation portion for separating blood cells from blood; and a development portion in which serum or plasma contained in the blood develops. The blood cell separation portion in this blood retention tool is the blood cell separation membrane according to the present invention. Since the blood retention tool of the present invention is provided with a blood cell separation membrane producing the above-described effect, it is particularly useful in remote clinical testing systems as described above, for example. The blood retention tool according to the present invention also can be used as a tool for retaining a blood specimen in which blood cells and serum/plasma are separated for the purpose of, for example, sending the specimen by mail or the like. Alternatively, the blood retention tool itself can be used as a blood testing tool for testing an analyte.

### Brief Description of Drawing

FIG. 1 is a sectional view showing an example of a blood retention tool according to the present invention.
FIG. 2 is a sectional view showing another example of a blood retention tool according to the present invention.
FIG. 3 is a sectional view showing still another example of a blood retention tool according to the present invention.
FIG. 4 is a sectional view showing still another example of a blood retention tool according to the present invention.
FIG. 5 is a sectional view showing still another example of a blood retention tool according to the present invention.
FIG. 6 is a sectional view showing still another example of a blood retention tool according to the present invention.
FIG. 7 is a sectional view showing still another example of a blood retention tool according to the present invention.
FIG. 8 is a sectional view showing still another example of a blood retention tool according to the present invention.
FIG. 9 is a sectional view showing still another example of a blood retention tool according to the present invention.
FIG. 10 is a sectional view showing still another example of a blood retention tool according to the present invention.

### Description of the Invention

As described above, the blood cell separation membrane according to the present invention includes a porous membrane for separating blood into blood cells and serum or plasma. The blood cell separation membrane is characterized in that the porous membrane contains at least one hemolysis inhibitor selected from the group consisting of valine, leucine, silk extracts ε-aminohexanoic acid, and tranexamic acid.

As the hydrophobic aminocarboxylic acid, a hydrophobic amino acid can be used, for example. More specifically, alanine, valine, leucine, isoleucine, or the like can be used. The proteins derived from silk (hereinafter also referred to as "silk extracts") refer to hydrolysates of fibroin.

Among the above-described hemolysis inhibitors, valine, leucine, ε-aminohexanoic acid, tranexamic acid, and silk extracts are preferable. These hemolysis inhibitors can produce an effect of separating blood into serum/plasma and blood cells highly efficiently, in addition to the effect of preventing hemolysis as described above. Although the mechanism is unknown, when the blood cell separation membrane contains at least one of these preferable hemolysis inhibitors, the speed at which blood cells penetrate into the blood cell separation membrane decreases, whereas the speed at which serum/plasma penetrates into the same increases.
Furthermore, even in the case where blood cells pass through the blood cell separation membrane, the speed at which the blood cells develop in the development portion decreases, whereas the speed at which serum or plasma develops in the same increases, for example. Accordingly, in the development portion, it is possible to make the distance by which the blood cells develop short and the distance by which the serum or plasma develops long (hereinafter such a distance is referred to simply as a "development distance"). That is, the above-described preferable hemolysis inhibitors can achieve conflicting effects, i.e., suppression of the blood cell development and promotion of the serum/plasma development, thereby increasing the developing amount of the serum or plasma only. Therefore, in the blood cell separation membrane containing the above-described preferable hemolysis inhibitor, blood can be separated into blood cells and serum or plasma more easily Furthermore, even in the case where blood cells pass through the blood cell separation membrane, the influence of the blood cell development on the collection rate of serum/plasma can be reduced, so that the collection rate of serum/plasma can be improved. Thus, the amount of blood to be supplied to the blood cell separation membrane may be smaller, so that the burden on the patient at the time of collecting blood can be reduced, for example.

Furthermore, among the above-described preferable hemolysis inhibitors, valine, tranexamic acid, and ε-aminohexanoic acid are particularly preferable. These hemolysis inhibitors can produce the above-described effect sufficiently even though their content (weight) may be about 1/5 to 1/10 of that of glycine as described above, for example.

The hemolysis inhibitors may be used alone or in combinations of at least two kinds thereof.

In the present invention, the content (weight) of the hemolysis inhibitor preferably is in the range from 1 mg to 50 mg, more preferably from 5 mg to 40 mg, and particularly preferably from 10 mg to 30 mg per unit volume (cm³) of the porous membrane, for example. When the content of the hemolysis inhibitor is at least 1 mg per unit volume (cm³) of the porous membrane, it is possible to prevent hemolysis sufficiently. It should be noted here that "the volume of the porous membrane" refers to the volume of the porous membrane including the volume of pores.

Specifically, in the case where the hemolysis inhibitor is valine, the content (weight) thereof preferably is in the range from 5 mg to 50 mg, more preferably from 15 mg to 45 mg, and particularly preferably from 30 mg to 40 mg per unit volume (cm³) of the porous membrane, for example.

In the case where the hemolysis inhibitor is leucine, the content (weight) thereof preferably is in the range from 1 mg to 30 mg, more preferably from 5 mg to 25 mg, and particularly preferably 10 mg to 20 mg per unit volume (cm³) of the porous membrane, for example.

In the case where the hemolysis inhibitor is tranexamic acid, the content (weight) thereof preferably is in the range from 5 mg to 50 mg, more preferably from 15 mg to 45 mg, and particularly preferably from 30 mg to 40 mg per unit volume (cm³) of the porous membrane, for example.

In the case where the hemolysis inhibitor is ε-aminohexanoic acid, the content (weight) thereof preferably is in the range from 5 mg to 50 mg, more preferably from 10 mg to 45 mg, and particularly preferably from 30 mg to 40 mg per unit volume (cm³) of the porous membrane, for example.

The blood separation membrane of the present invention further may contain additives such as pullulan and BSA, in addition to the hemolysis inhibitor. By using such additives and the hemolysis inhibitor in combination, the effect of preventing hemolysis can be further improved.

Although it is preferable that the hemolysis inhibitor is contained over the entire area of the blood cell separation membrane, the hemolysis inhibitor may be contained in, for example, at least one surface of the blood cell separation membrane, in particular, the surface of the blood cell separation membrane to which blood is supplied.

In general, it is desirable that blood cells cannot pass through the porous membrane easily. For example, it is preferable that the porous membrane has pores through which blood cells cannot pass. In the present invention, the "pores through which blood cells cannot pass" are not limited to the pores with smaller sizes than spherical diameters of blood cells, but may be pores through which blood cells eventually cannot pass regardless of the mechanism of preventing blood cells from passing through the pores. Therefore, the pores through which blood cells cannot pass may include pores larger than the spherical diameters of blood cells. Moreover, according to the blood cell separation membrane of the present invention, for example, even when blood cells have passed through the separation membrane as described above, the hemolysis inhibitor can increase the developing speed of serum or plasma and decrease the developing speed of blood cells. In this case, it is not necessary for the porous membrane to prevent blood cells from passing therethrough completely to retain all the blood cells therein, and some of the blood cells may pass through the porous membrane, for example.

More specifically, it is preferable that the porous membrane has pores with a pore size of 0.1 µm to 20 µm, more preferably 1 µm to 10 µm, and particularly preferably 2 µm to 8 µm.

There is no particular limitation regarding the porous membrane, and various materials that have been used conventionally for blood cell separation can be used as the porous membrane. More specifically, it is possible to use a glass filter or an asymmetric porous membrane with a pore size distribution in which an average pore size varies so as to be reduced continuously or discontinuously in the thickness direction, for example.

As the glass filter, those having a low fiber density are preferable, for example, and commercially available glass filters such as a product named "AP25" manufactured by Millipore Corporation can be used, for example.

In the case where the asymmetric porous membrane is used as the porous membrane, the pore size varies in the thickness direction. Thus, for example, as blood moves in the thickness direction inside the porous membrane, fewer and fewer blood cells can pass through the pores, and the blood cells are retained when they reach the portion with pores through which they cannot pass. Therefore, the clogging hardly occurs and blood cells can be separated quickly and easily. In the present invention, the "average pore size varies so as to be reduced discontinuously" means that the average pore size may vary, for example, so as to be reduced in a stepwise manner.

In the asymmetric porous membrane, it is preferable that the maximum pore size is in the range from 10 µm to 300 µm and the minimum pore size is in the range from 0.1 µm to 30 µm. It is more preferable that the maximum pore size is in the range from 100 µm to 200 µm and the minimum pore size is *µm* the range from 1 µm to 10 µm. It is particularly preferable that the maximum pore size is in the range from 150 µm to 200 µm and the minimum pore size is in the range from 1 µm to 5 µm.

The material of the asymmetric porous membrane is not particularly limited, and may be, for example, a resin such as polyester, polysulfone, polyethersulfone, polycarbonate, cellulose acetate, polyamide, polyimide, polystyrene, or the like. The material is not limited to only one of them, and two or more of them may be used in combination. Among them, polysulfone and polyethersulfone are preferable, and polyethersulfone is particularly preferable, for example.

The asymmetric porous membrane may be formed using the above-described various resins. Alternatively, commercially available asymmetric porous membranes, e.g., a product named "BTS-SP" manufactured by U.S. Filter Corporation and a product named "Primecare S/G" manufactured by Spectral Diagnostics, Inc., may be used as the asymmetric porous membrane.

The size of the blood cell separation membrane can be determined as appropriate according to the amount of blood to be supplied or the like, for example. Specifically, when the blood to be supplied is 120 µl, the size (length × width × thickness) of the blood cell separation membrane is, for example, in the range between 4 mm × 1.5 mm × 50 µm and 50 mm × 20 mm × 2000 µm inclusive, preferably between 5 mm × 3 mm × 75 µm and 30 mm × 15 mm × 1250 µm inclusive, and more preferably between 10 mm × 5 mm × 90 µm and 20 mm × 12 mm × 1100 µm inclusive. It should be noted that the "length" refers to the dimension in the longitudinal direction of the blood cell separation membrane, and the "width" refers to the dimension in the width direction of the same. The same applies hereinafter.

When a glass filter is used as the blood cell separation membrane, the thickness thereof is, for example, in the range from 200 µm to 2000 µm, preferably from 500 µm to 1000 µm. Furthermore, when the blood to be supplied is 120 µl, the size (length × width × thickness) of the glass filter is, for example, in the range between 4 mm × 1.5 mm × 100 µm and 50 mm × 20 mm × 2000 µm inclusive, preferably between 5 mm × 3 mm × 200 µm and 30 mm × 15 mm × 1250 µm inclusive, and more preferably between 10 mm × 5 mm × 250 µm and 20 mm × 12 mm × 1100 µm inclusive.

When an asymmetric porous membrane is used as the blood cell separation membrane, the thickness thereof is, for example, in the range from 50 µm to 400 µm, preferably from 100 µm to 350 µm. Furthermore, when the blood to be supplied is 40 µl, the size (length width × thickness) of the asymmetric porous membrane is, for example, in the range between 4 mm × 1.5 mm × 50 µm and 50 mm × 20 mm × 400 µm inclusive, preferably between 5 mm × 3 mm × 100 µm and 30 mm × 15 mm × 350 µm inclusive, and more preferably between 10 mm × 5 mm × 200 µm and 20 mm × 12 mm × 300 µm inclusive.

The method for producing the blood cell separation membrane of the present invention is not particularly limited. For example, the blood cell separation membrane can be produced by immersing the porous membrane in a dispersion or a solution of the hemolysis inhibitor and then drying the porous membrane, or by dropping the dispersion or the like on the porous membrane, allowing the dispersion to penetrate into the porous membrane, and then drying the porous membrane.

The concentration of the hemolysis inhibitor in the dispersion or the solution is, for example, in the range from 0.1 wt% to 5 wt%, preferably from 0.5 wt% to 4 wt%, and more preferably from 2 wt% to 3 wt%.

The porous membrane may be treated so as to be provided with hydrophilicity by being immersed in a treatment solution of, for example, a hydrophilic polymer such as hydroxypropylcellulose (HPC), polyvinyl alcohol (PVA), or carboxymethylcellulose (CMC) before the hemolysis inhibitor is added thereto, because this allows whole blood to penetrate into the porous membrane rapidly. The concentration of the hydrophilic polymer in the treatment solution is, for example, in the range from 0.1 wt% to 50 wt%, and the treatment time is, for example, in the range from 0.1 to 24 hours. As a solvent of the treatment solution, for example, water, various organic solvents, or the like can be used. Examples of the organic solvents include alcohols such as ethanol.

Next, as described above, the blood retention tool according to the present invention includes: a blood cell separation portion for separating blood cells from blood; and a development portion in which serum or plasma contained in the blood develops. The blood retention tool is characterized in that the blood cell separation portion is the blood cell separation membrane according to the present invention.

Embodiments of the blood retention tool according to the present invention include: Embodiment A-1 directed to a laminate-type blood retention tool in which a blood cell separation portion is laminated on a development portion; Embodiments A-2 and A-3, directed to a single-layer blood retention tool in which an asymmetric porous membrane includes a development portion and a blood cell separation portion; and an embodiment directed to a single-layer blood retention tool in which a blood separation membrane includes a blood cell separation portion and a development portion. In the following, Embodiments A-1, A-2, and A-3 will be described specifically.

### (Embodiment A-1)

The present embodiment is directed to a blood retention tool configured so that: a blood cell separation portion is a blood cell separation membrane according to the present invention; a development portion is composed of a porous membrane; and the blood cell separation membrane is laminated on the development portion (the development porous membrane). In this embodiment, a surface of the blood cell separation membrane serves as a blood supply portion.

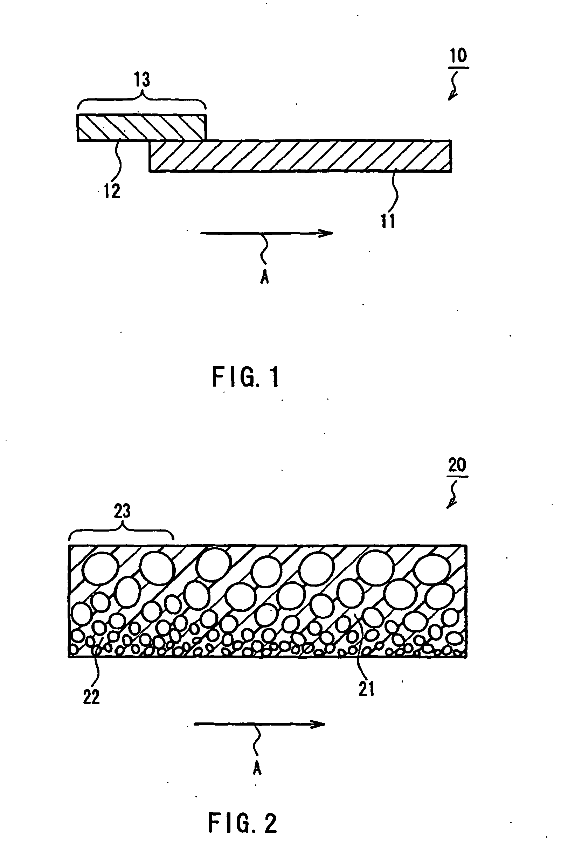

FIG. 1 is a sectional view showing an example of such a blood retention tool. As shown in FIG. 1, in this blood retention tool 10, a blood cell separation portion 12 is laminated on one end of a development portion 11, and a surface of the blood cell separation portion 12 serves as a blood supply portion 13. In FIG. 1, an arrow A indicates the direction in which serum/plasma contained in blood develops (the same applies to FIGs. 2 to 10).

As the development porous membrane composing the development portion 11, a filter paper, a cellulose acetate membrane, a porous membrane, or a glass fiber membrane can be used, for example. Examples of the material of the porous membrane include resins such as polyester, polysulfone, polyethersulfone, polycarbonate, cellulose acetate, polyamide, polyimide, and polystyrene. Among them, polyethersulfone and polycarbonate are preferable. Also, an asymmetric porous membrane as described above can be use as the development porous membrane. In the case where the asymmetric porous membrane is used as the development portion, even when blood cells pass through the blood cell separation portion, blood cell separation can be carried out further in the development portion by the pore structure of the asymmetric porous membrane. Note here that the above-described porous membranes may be used alone or in combinations of at least two kinds thereof. Among them, a filter paper, a cellulose acetate membrane, a nitrocellulose membrane, a polysulfone porous membrane, a polyester porous membrane, and a polycarbonate porous membrane are preferable, and a filter paper, a polysulfone porous membrane, and a polyester porous membrane are particularly preferable, for example. Moreover, as in the case of the above-described blood cell separation membrane, the development porous membrane also may be treated so as to be provided with hydrophilicity, because this further promotes the development of serum or plasma.

The average pore size of pores of the development porous membrane is not particularly limited as long as serum or plasma is allowed to develop by capillary action, for example. However, it is preferable that the average pore size is in the range from 0.1 µm to 300 µm, more preferably from 1 µm to 100 µm, and particularly preferably from 2 µm to 50 µm. The size of this development porous membrane can be determined as appropriate according to the amount of blood to be supplied or the like, for example. When the blood to be supplied is 40 *µl,* the size (length × width × thickness) of this development porous membrane is, for example, in the range between 4 mm × 1.5 mm × 50 µm and 50 mm × 20 mm × 400 µm inclusive, preferably between 5 mm × 3 mm × 100 µl and 30 mm × 15 mm × 350 µm inclusive, and more preferably between 10 mm × 5 mm × 200 µm and 20 mm × 12 mm × 300 µm inclusive.

. Note here that the hemolysis inhibitor may be contained not only in the blood cell separation membrane but also in the development porous membrane.

The combination of the blood cell separation membrane and the development porous membrane is not particularly limited, and preferable examples thereof include the combination of a glass filter as the blood cell separation membrane and an asymmetric porous membrane as the development porous membrane and the combination of an asymmetric porous membrane as the blood cell separation membrane and a nitrocellulose membrane as the development porous membrane.

In the blood retention tool according to the present embodiment can be produced by laminating the blood cell separation membrane on the development porous membrane. The lamination may be carried out, for example, by merely placing the blood cell separation membrane on the development porous membrane or by positioning the blood cell separation membrane with respect to the development porous membrane and then bonding or attaching by pressure the end portions of these membranes.

Preferably, the development porous membrane is supported by a supporter. This allows a blood testing tool with a sufficient strength to be obtained regardless of the strength of the development porous membrane and also enables easy handling. As the material for forming the supporter, for example, plastic such as polystyrene, polyethylene terephthalate (PET), polyvinyl chloride, an acrylic resin, acrylonitrile-butadiene-styrene copolymer (ABS), or the like can be used. The material is not limited to only one of them, and two or more of them may be used in combination. When the blood retention tool is to be subjected to the measurement directly by the optical method or the like as will be described later, it is preferable that the supporter is optically transparent. In this case, the supporter may be made of polystyrene, PET, an acrylic resin, or the like, for example.

Next, an example of preparing a serum sample or a plasma sample by adding blood to the blood retention tool 10 will be described with reference to FIG. 1.

First, blood is dropped on the blood supply portion 13 on the surface of the blood cell separation portion 12. The blood moves in the thickness direction inside the blood cell separation portion 12, during which the blood is separated into blood cells and serum/plasma. The serum/plasma thus separated reaches the development portion 11 and then develops in a direction parallel to the surface (the direction indicated by the arrow Ain FIG. 1 and hereinafter referred to simply as a "surface direction") of the development portion 11 by capillary action. In the above process, according to the blood retention tool of the present invention, hemolysis accompanying the blood cell separation can be prevented because the hemolysis inhibitor is contained in the blood cell separation portion 12. Accordingly, the possibility that the serum/plasma sample obtained might contain components of the blood cells can be reduced.

When collecting the serum/plasma sample that has developed, the blood retention tool 10 may be dried by air drying, natural drying, or the like. Thereafter, the development portion 11 may be removed from the blood retention tool 10, and then only the portion in which the serum or the plasma has developed may be cut out from the development portion 11.

A cut piece obtained by cutting or the like is put, for example, in a test tube and an extractant is added thereto, which then is left, thus extracting and collecting serum or plasma. The extractant is not particularly limited as long as it can extract serum or plasma and does not affect the detection of an analyte in the serum or plasma. As the extractant, for example, a buffer solution, a physiological salt solution, purified water, a protein solution, or the like or a mixture thereof may be used. Examples of the buffer solution include various buffer solutions containing phosphoric acid, citric acid, hydrochloric acid, acetic acid, or the like, and the pH of the buffer solution is, for example, in the range from 6 to 8. The amount of the extractant to be added is not particularly limited, and can be determined as appropriate according to the size of the cut piece or the like. The amount is, for example, 1 to 1000 times the volume of the cut piece. Furthermore, the time for an extracting process is not particularly limited and is, for example, in the range from 1 to 300 minutes.

Note here that after the serum or the plasma has developed, the serum or the plasma can be collected by cutting out the development portion and then subjecting the cut piece to centrifugation directly.

Using the solution thus collected, an analyte in the serum or plasma can be measured.

Alternatively, it is also possible to analyze the serum or plasma retained in the development portion 11 in this blood retention tool 10 without collecting it using the extractant. In this case, an analytical reagent may be provided in the development portion 11 beforehand to form a reagent portion. Examples of the method of providing the reagent in the development portion 11 include a printing method, an impregnation method, and a spraying method.

The analytical reagent is not particularly limited, and can be determined as appropriate according to the kind of an analyte. Examples of the components of the reagent include various enzymes, buffer materials such as phosphate and carbonate, and color developing agents. More specifically, when the analyte is glucose, the reagent may contain glucokinase, glucose-6-phosphate dehydrogenase, β-NADP, ATP, a buffer solution, and the like, for example.

Furthermore, when a plurality of reagents are provided in the development portion 11 so as to be parallel to the direction in which the serum or the plasma moves, multiple items can be analyzed in a single blood retention tool. In this case, in order to prevent reagents for the multiple items from being mixed, it is preferable to provide boundary layers between the respective reagent portions by, for example, impregnation of a hydrophobic resin solution.

In the blood retention tool 10 configured as above, when blood is supplied to the blood retention tool 10 to cause serum or plasma to develop as described above, various analytes react with detection reagents, respectively, in the development portion 11. The analysis can be conducted easily by detecting, for example, color developed through these reactions according to an electrochemical method, an optical method (including visual observation), or the like.

### (Embodiment A-2)

The present embodiment is directed to a blood retention tool configured so that: a blood cell separation portion is an asymmetric porous membrane; the asymmetric porous membrane further includes a development portion; and the blood cell separation portion and the development portion are arranged along the surface direction of the asymmetric porous membrane so that the blood cell separation portion is on a side from which blood is supplied and the development portion is on a downstream side in the developing direction of the blood supplied. That is, a single asymmetric porous membrane includes the blood cell separation portion and the development portion arranged along the surface direction, so that blood cell separation and development of serum or plasma can be performed within the single asymmetric porous membrane.

FIG. 2 is a sectional view showing an example of such a blood retention tool. As shown in FIG. 2, this blood retention tool 20 is composed of an asymmetric porous membrane with a pore size distribution in which an average pore size varies so as to be reduced continuously or discontinuously in a thickness direction. The portion on an upstream side in the direction indicated by the arrow A serves as a blood cell separation portion 22, and the portion on a downstream side in the direction indicated by the arrow A serves as a development portion 21. Furthermore, the surface of the blood cell separation portion 22 serves as a blood supply portion 23.

In the asymmetric porous membrane, the maximum pore size is, for example, in the range from 10 µm to 300 µm, preferably from 50 µm to 150 µm, and the minimum pore size is, for example, in the range from 0.1 µm to 30 µm, preferably from 1 µm to 10 µm.

The size of the blood retention tool 20 is not particularly limited as in the above, and can be determined as appropriate according to the amount of a blood specimen to be supplied or the like. When the whole blood to be supplied is about 100 µl, the size (length × width × thickness) of the blood retention tool 20 as a whole is, for example, in the range between 4 mm × 1.5 mm × 50 µm and 60 mm × 20 mm × 400 µm inclusive, preferably between 5 mm × 3 mm × 100 µm and 30 mm × 15 mm × 350 µm inclusive, and more preferably between 10 mm × 5 mm × 200 µm and 20 mm × 12 mm × 300 µm inclusive. It should be noted that the "length" refers to the dimension in the longitudinal direction of the blood retention tool, and the "width" refers to the dimension in the width direction of the same.

In the blood retention tool 20 configured as above, the blood cell separation portion 22 and the development portion 21 need not be separated explicitly with a boundary line, for example. In the blood retention tool 20, when blood is dropped on the blood supply portion 23, the blood moves in the thickness direction, during which the blood also develops in the surface direction (the direction indicated by the arrow A in FIG. 2) while being separated into blood cells and serum or plasma. The size (length × width) of the blood supply portion 23 is, for example, in the range between 2 mm × 1.5 mm and 15 mm × 20 mm inclusive, preferably between 4 mm × 3 mm and 10 mm × 15 mm inclusive, and more preferably between 4 mm × 5 mm and 6 mm × 12 mm inclusive. In general, the region that lies in the thickness direction of this blood supply portion 23 corresponds to the blood cell separation portion 22.

As described above, this blood retention tool 20 is composed of an asymmetric porous membrane having a single layer structure. Thus, for example, a solution containing the hemolysis inhibitor may be contained only in a portion serving as the blood cell separation portion 22. Alternatively, the entire asymmetric porous membrane may be immersed in the solution so that the entire area of the porous membrane is impregnated with the solution.

Next, an example of preparing a serum sample or a plasma sample by adding blood to the blood retention tool 20 will be described with reference to FIG. 2.

First, blood is dropped on the blood supply portion 23. The blood moves in the thickness direction inside the blood cell separation portion 22, during which blood cells are separated from the blood. Serum or plasma moves in the surface direction (the direction indicated by the arrow A in FIG. 2) and develops in the development portion 21 by capillary action. After that, the serum or plasma can be collected from the development portion 21 in the same manner as in Embodiment A-1.

### (Embodiment A-3)

In the blood retention tool composed of an asymmetric porous membrane including both the blood cell separation portion and the development portion as described in Embodiment A-2, it is preferable that a blood cell blocking portion further is provided, for example, at the boundary between the blood cell separation portion and the development portion in order to improve the efficiency of blood cell separation further. More specifically, a blood cell blocking portion including only pores through which blood cells cannot pass may be formed between the blood cell separation portion and the development portion so as to extend in the width direction of the asymmetric porous membrane. In the blood retention tool provided with the blood cell blocking portion, with respect to the direction in which blood develops when it is supplied thereto, a portion downstream from the blood cell blocking portion serves as a development portion, and the blood cell blocking portion and a portion upstream from the blood cell blocking portion serve as a blood cell separation portion. The blood retention tool configured as above is advantageous in that, for example, even when blood cells contained in the blood move not only in the thickness direction but also in the surface direction, it is possible to prevent the blood cells from entering the development portion reliably by the blood cell blocking portion so that only serum or plasma develops in the development portion. Preferably, a groove is formed so as to extend in the width direction of the asymmetric porous membrane, and a portion between the bottom of the groove and a part of the asymmetric porous membrane surface corresponding to the bottom serves as the blood cell blocking portion. Unless otherwise stated, conditions (the size, the material, etc.) of the blood retention tool according to the present embodiment are the same as those of the blood retention tool according to Embodiment A-2.

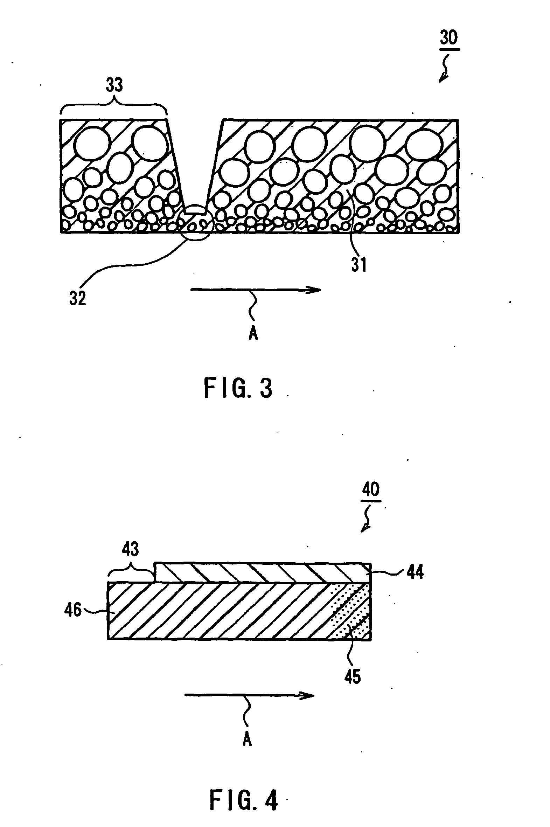

FIG. 3 is a sectional view showing an example of such a blood retention tool. As shown in FIG. 3, this blood retention tool 30 is composed of an asymmetric porous membrane having a single layer structure similar to the blood retention tool 20 of Embodiment A-2, and a groove further is formed so as to extend in the width direction of the asymmetric porous membrane. A portion between the bottom of the groove and a part of the asymmetric porous membrane surface corresponding to the bottom serves as a blood cell blocking portion 32. The blood cell blocking portion 32 and a portion upstream therefrom in the direction indicated by the arrow A serve as a blood cell separation portion, and a portion downstream from the blood cell blocking portion 32 in the direction indicated by the arrow A serves as a development portion 31. Furthermore, the surface of the blood cell separation portion with larger pores serves as a blood supply portion 33.

In the asymmetric porous membrane, the size of the pores in the blood cell blocking portion 32 is, for example, in the range from 0.1 µm to 50 µm, preferably from 1 µm to 30 µm.

The size of the blood retention tool 30 is not particularly limited as in the above, and can be determined as appropriate according to the amount of a blood specimen to be supplied or the like. When the whole blood to be supplied is about 100 µl, the size (length × width × thickness) of the blood cell blocking portion 32 is, for example, in the range between 0.1 mm × 1.5 mm × 10 µm and 10 mm × 20 mm × 400 µm inclusive, preferably between 0.2 mm × 3 mm × 15 µm and 8 mm × 15 mm × 350 µm inclusive, and more preferably between 0.3 mm × 5 mm × 20 µm and 6 mm × 12 mm × 300 µm inclusive.

The groove can be formed, for example, by compression of a part of the surface of the asymmetric porous membrane. For instance, the groove can be formed by compression through rolling of a disk-shaped roller or by compression using a cutting tool with a dull edge to a degree causing no cut. When the groove is formed by compression, the blood cell blocking portion may include large pores of the asymmetric porous membrane. However, since the pores are deformed or crushed after compression, the blood cells are thus prevented from passing through the blood cell blocking portion.

Alternatively, the groove may be formed, for example, by cutting off a part of the porous membrane using a cutting tool such as a cutter. In this case, the size of the groove is the same as in the case described above.

Next, as specific examples of the blood retention tool according to the present invention, those that can be used as a blood testing tool will be described by way of embodiments with reference to FIGs. 4 to 10. However, it should be noted that the blood retention tool of the present invention is by no means limited to the specific examples given below.

### (Embodiment B-1)

FIG. 4 is a sectional view showing a blood retention tool according to a first embodiment (B-1). As shown in FIG. 4, a blood retention tool 40 includes a blood separation membrane 46 and a supporter 44. At one end of the blood separation membrane 46, a reagent portion 45 containing an analytical reagent is formed. On one surface of the blood separation membrane 46, the supporter 44 is laminated so that the other end (i.e., the end opposite to the end provided with the reagent portion 45) of the blood separation membrane 46 is exposed. The exposed portion serves as a blood supply portion 43.

In the blood retention tool 40 configured as above, when blood is dropped on the blood supply portion 43, the blood moves in the direction indicated by the arrow A inside the blood separation membrane 46, during which the blood is separated into blood cells and serum, for example. Since the blood separation membrane 46 contains a hemolysis inhibitor as described above, it is possible to separate the blood into blood cells and serum while preventing the hemolysis of the blood cells. The serum separated moves inside the blood separation membrane 46 to develop in the reagent portion 45, where an analyte contained in the serum reacts with the analytical reagent. The analysis can be conducted by detecting this reaction according to an electrochemical method or an optical method (including visual observation), for example.

### (Embodiment B-2)

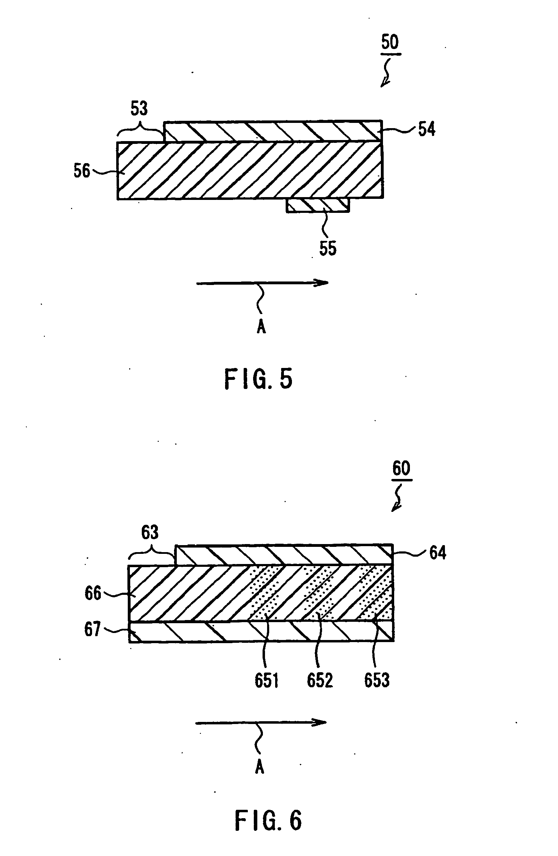

FIG. 5 is a sectional view showing a blood retention tool according to a second embodiment (B-2). Unless otherwise stated, the present embodiment is the same as the first embodiment (B-1). This applies to third to sixth embodiments (B-3 to B-6) described later.

As shown in FIG. 5, a blood retention tool 50 includes a blood separation membrane 56, a reagent layer 55 containing an analytical reagent, and a supporter 54. On one surface of the blood separation membrane 56, the supporter 54 is laminated so that one end of the blood separation membrane 56 is exposed. This exposed portion serves as a blood supply portion 53. Furthermore, on the other surface (i.e., the surface opposite to the surface provided with the supporter 54) of the blood separation membrane 56, the reagent layer 55 is laminated at an end portion that is on the side opposite to the blood supply portion 53 side.

In the blood retention tool 50 configured as above, serum separated in the blood separation membrane 56 develops in the reagent layer 55 laminated on the blood separation membrane 56, so that an analyte contained in the serum reacts with the analytical reagent contained in the reagent layer 55, for example.

### (Embodiment B-3)

FIG. 6 is a sectional view showing a blood retention tool according to a third embodiment (B-3). As shown in FIG. 6, a blood retention tool 60 includes a blood separation membrane 66 and supporters 64 and 67. The supporter 67 is laminated on one surface of the blood separation membrane 66, and the supporter 64 is laminated on the other surface of the blood separation membrane 66 so that one end of the blood separation membrane 66 is exposed. This exposed portion serves as a blood supply portion 63. The blood separation membrane 66 includes three reagent portions, each containing an analytical reagent. The first reagent portion 651, the second reagent portion 652, and the third reagent portion 653 are formed in this order along the direction indicated by the arrow A in FIG. 6. The first reagent portion 651 contains a first analytical reagent, the second reagent portion 652 contains a second analytical reagent, and the third reagent portion 653 contains a third analytical reagent.

In the blood retention tool 60 configured as above, for example, serum separated in the blood separation membrane 66 first develops in the first reagent portion 651, where an analyte contained in the serum reacts with the first analytical reagent. A reaction solution obtained through this reaction further develops in the second reagent portion 652 to react with the second analytical reagent. Then, a reaction solution obtained through this reaction finally develops in the third reagent portion 653 to react with the third analytical reagent. This reaction can be detected in the manner described above.

The blood retention tool 60 with such a configuration is suitable in the case where an analyte is to be measured utilizing a multistep reaction, and the first reagent portion 651, the second reagent portion 652, and the third reagent portion 653 may be provided with appropriate reagents according to the reaction sequence in the multistep reaction.

### (Embodiment B-4)

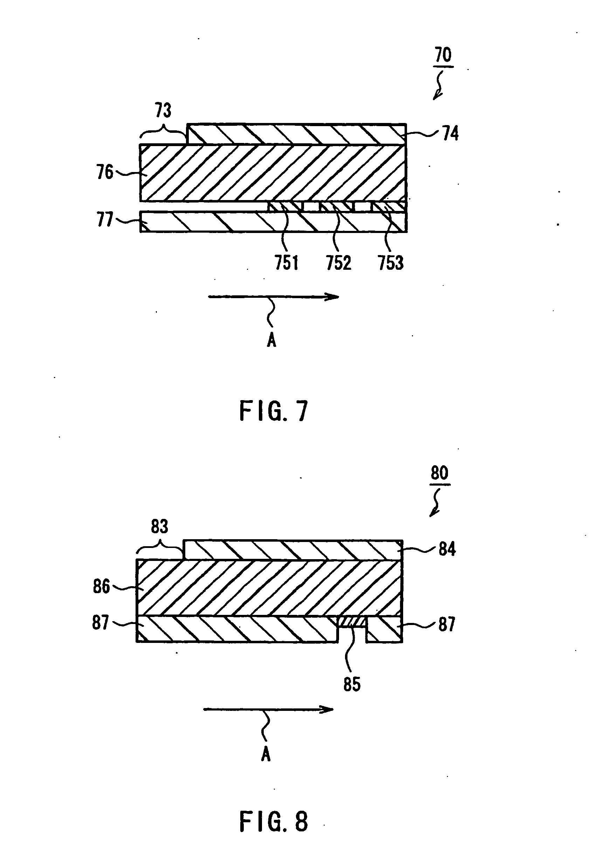

FIG. 7 is a sectional view showing a blood retention tool according to a fourth embodiment (B-4). As shown in FIG. 7, a blood retention tool 70 includes a blood separation membrane 76, three reagent layers (751, 752, and 753), each containing an analytical reagent, and supporters 74 and 77. On one surface of the blood separation membrane 76, the supporter 74 is laminated so that one end of the blood separation membrane 76 is exposed. This exposed portion serves as a blood supply portion 73. The first reagent layer 751, the second reagent layer 752, and the third reagent layer 753 are laminated on the other surface of the blood separation membrane 76 so as to be arranged in this order along the direction indicated by the arrow Ain FIG. 7. The supporter 77 further is laminated on this surface of the blood separation membrane 76 via these reagent layers. The first reagent layer 751 contains a first analytical reagent, the second reagent layer 752 contains a second analytical reagent, and the third reagent layer 753 contains a third analytical reagent.

In the blood retention tool 70 configured as above, for example, serum separated in the blood separation membrane 76 develops in the first reagent layer 751, the second reagent layer 752, and the third reagent layer 753 laminated on the blood separation membrane 76. Analytes contained in the serum respectively react with the analytical reagents contained in the respective reagent layers (751,752, and 753). Thus, multiple test items can be analyzed in a single blood retention tool by, for example, providing the reagent layers with reagents corresponding to the respective items.

### (Embodiment B-5)

FIG. 8 is a sectional view showing a blood retention tool according to a fifth embodiment (B-5).

As shown in FIG. 8, a blood retention tool 80 includes a blood separation membrane 86, a reagent layer 85 containing an analytical reagent, and supporters 84 and 87. On one surface of the blood separation membrane 86, the supporter 84 is laminated so that one end of the blood separation membrane 86 is exposed. This exposed portion serves as a blood supply portion 83. On the other surface of the blood separation membrane 86, the supporter 87 having a through hole is laminated, and the reagent layer 85 is laminated on the portion corresponding to the through hole.

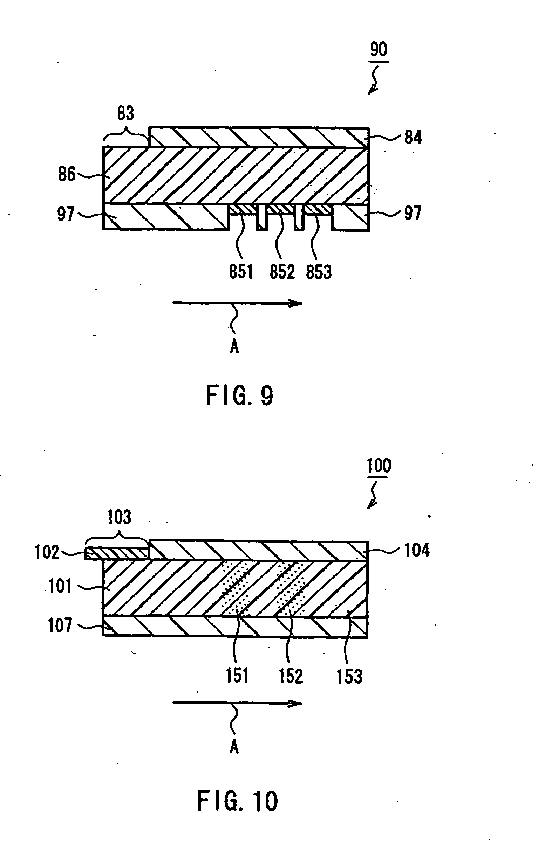

Note here that, as shown in the sectional view of FIG. 9, three through holes may be provided on a supporter 97so as to be arranged along the direction indicated by the arrow A, and reagent layers (851, 852, and 853) may be provided at portions on the surface of the blood separation membrane 86 corresponding to the respective through holes. This blood retention tool 90 also is suitable for analysis of multiple test items, similar to the blood retention tool according to the fourth embodiment (B-4). In FIG. 9, the same components as those in FIG. 8 are given the same reference numerals.

### (Embodiment B-6)

FIG. 10 is a sectional view showing a blood retention tool according to a sixth embodiment (B-6).

As shown in FIG. 10, a blood retention tool 100 includes a blood separation membrane 102, a development layer 101, and supporters 104 and 107. The supporter 107 is laminated on one surface of the development layer 101, and the supporter 104 is laminated on the other surface of the development layer 101 so that one end of the development layer 101 is exposed. The blood separation membrane 102 is laminated on this exposed portion. The surface of the blood separation membrane 102 serves as a blood supply portion 103. In the development layer 101, a first reagent portion 151 and a second reagent portion 152, each containing an analytical reagent, are formed in this order along the direction indicated by the arrow Ain FIG. 6. The second reagent portion 152 also serves as a first detecting portion, and a portion downstream from the second reagent portion (the first detecting portion) 152 in the direction indicated by the arrow A serves as a second detecting portion 153.

In the following, an example of a method of analyzing a component of serum through an antigen-antibody reaction using the blood retention tool 100 will be described. In the first reagent portion 151, a labeled first antibody against an analyte (an antigen) and a labeled third antibody against a second antibody that will be described later are provided. In the second reagent portion 152, an unlabeled second antibody against the antigen is immobilized. As the labels of the labeled first antibody and the labeled third antibody, colored latex particles can be used.

First, blood is dropped on the blood supply portion 103. The blood passes through the blood separation membrane 102, during which serum is separated from the blood. The serum thus separated moves in the direction indicated by the arrow A inside the development layer 101. The serum first develops in the first reagent portion 151, where the antigen contained in the serum forms a complex with the labeled first antibody through an antigen-antibody reaction. The serum, which contains the complex, the labeled first antibody not bound to the antigen, and the labeled third antibody, further develops in the second reagent portion 152. In the second reagent portion 152, the complex and the labeled third antibody are bound to the unlabeled second antibody immobilized in the second reagent portion 152 through an antigen-antibody reaction and thus are captured in the second reagent portion 152. Then, the serum, which contains the labeled first antibody not bound to the antigen and also the complex and the labeled third antibody that are not captured in the first detecting portion (the second reagent portion) 152, further develops in the second detecting portion 153.

After that, the measurement can be carried out in the following manner. First, in the first detecting portion (the second reagent portion) 152, the captured complex of the antigen and the labeled first antibody is detected. In the first detecting portion 152, the captured labeled third antibody further is detected, and in the second detecting portion 153, the labeled third antibody not captured in the second reagent portion 152 is detected. Note here that the above-described detections can be carried out by measuring an absorbance at a wavelength specific to each of the labels (the colored latex particles) of the labeled first antibody and the labeled third antibody. Based on the result of the detections of the labeled third antibody, the capturing efficiency of the second reagent portion 152 is determined. Then, using the thus-determined capturing efficiency and the result of detection showing the apparent amount of the complex, the actual amount of the complex (i.e., the amount of the antigen) is calculated. By measuring the labeled third antibody as well, the correction using the capturing efficiency becomes possible. Thus, according to the present embodiment, the accuracy of analyzing an analyte (an antigen) can be improved.

### EXAMPLES

### (Example 1 and Comparative Example 1)

A blood retention tool according to Embodiment A-1 as shown in FIG. 1 was produced. Blood cell separation was carried out using this tool, and the presence or absence of hemolysis was determined. In the following, unless otherwise stated, the concentration (%) of a hemolysis inhibitor in a hemolysis inhibitor solution is represented by a percentage (unit: (w/v)%) of the weight (unit: g) of the hemolysis inhibitor with respect to the volume (100 ml) of a solvent.

### (Development porous membrane)

A commercially available asymmetric porous membrane (product name "Primecare S/G": Spectral Diagnostics, Inc.) made of polyethersulfone was washed under the following conditions and used as a development porous membrane. This porous membrane had a thickness of 290 µm, a length of 20 mm, and a width of 6 mm. In this porous membrane, the maximum pore size was 200 µm and the minimum pore size was 2 µm. The washing of the asymmetric porous membrane was carried out in the following manner. First, the asymmetric porous membrane was immersed in purified water and left for 10 minutes, after which the purified water was replaced with fresh purified water. This operation was carried out 5 times in total. Thereafter, the asymmetric porous membrane was dried by air drying for half a day, and then further dried in a desiccator for half a day. The asymmetric porous membrane then was used as a development porous membrane.

### (Blood separation membrane)

Hemolysis inhibitors were dissolved in solvents (distilled water) beforehand to prepare hemolysis inhibitor solutions shown in Table 1 below. Then, 30 µl of each of the hemolysis inhibitor solutions with various concentrations shown in Table 1 was dropped on a commercially available glass filter (product name "AP25": Millipore Corporation) having a thickness of 1200 µm, a length of 12 mm, and a width of 6 mm, which then was dried. The dried glass filter was used as a blood separation membrane. Note here that the silk extract shown below was a product named "Silk Extract" manufactured by FUKUI KINU SHOJI CO, LTD.

### (Method for producing blood retention tool)

A PET film having a length of 50 mm, a width of 6 mm, and a thickness of 180 µm was provided as a supporter. The development porous membrane was bonded to this PET film with an adhesive. Furthermore, the glass filter was disposed on one end of the development porous membrane as shown in FIG. 1. Then, a PET film (having a thickness of 100 µm) serving as a cover was disposed on the surface of the development porous membrane on which the glass filter was not disposed. The respective laminates obtained in the above-described manner were used as blood retention tools (Examples 1-1 to 1-8).

On the other hand, a blood retention tool according to Comparative Example 1 was produced in the same manner as in Example 1, except that a 5% glycine solution was contained in the glass filter.

### (Hemolysis of blood cells)

On the glass filter surface of each of the thus-obtained blood retention tools, 100 µl of whole blood (hematocrit: 38%) was dropped to cause the separation of blood cells and plasma and the development of the plasma. Then, after a lapse of 2 minutes, the presence or absence of hemolysis of the blood cells was determined. Note here that the plasma was allowed to develop until a tip of the development portion (the right end portion of the development portion 11 in FIG. 1) was impregnated with the plasma thoroughly. Whether or not the hemolysis of the blood cells occurred was judged according to whether the plasma that had developed was colored red with Hb contained in the blood cells.

**(Table 1)**

| | hemolysis inhibitor solution | hemolysis |
|---|---|---|
| Example 1-1 | 5% valine | none |
| 1-2 | 2% leucine | none |
| 1-3 | 5% ε-aminohexanoic acid | none |
| 1-4 | 5% tranexamic acid | none |
| 1-5 | 5% silk extract | none nonc |
| Comparative Example 1 | 5% glycine | occurred |

As shown in Table 1, hemolysis occurred in the blood retention tool according to Comparative Example 1. In contrast, in the blood retention tools according to Example 1 (1-1 to 1-5), the hemolysis was prevented from occurring, while the concentrations of the hemolysis inhibitor solutions contained in the respective blood retention tools were the same as or lower than that in the blood retention tool according to Comparative Example 1.

### (Example 2 and Comparative Example 2)

Blood retention tools were produced in the same manner as in Example 1, except that various hemolysis inhibitors shown in Table 2 below respectively were contained as hemolysis inhibitors. Using these blood retention tools, the development of blood cells was determined.

### (Measurement of development distance of blood cells)

As the development distance of blood cells, the distance from the end of the development porous membrane on the blood dropping side (the left end portion of the development portion 11 in FIG. 1) to a leading end of the blood cells that had developed was measured at the point in time when the plasma reached the tip of the development portion (the right end portion of the development portion 11 in FIG. 1).

**(Table 2)**

| | hemolysis inhibitor | | | development distance of blood cells |
|---|---|---|---|---|
| | type | concentration | content per glass filter | |
| | | (%) | (mg/cm³) | m/m) |
| Ex. 2-1 | valine | (%) 5 | 40 | 3 |
| 2-2 | leucine | 2 | 16 | 4 |
| 2-3 | ε-aminohexanoic acid | 5 | 40 | 6 |
| 2-4 | tranexamic acid | 5 | 40 | 4 |
| 2-5 | silk extract | 5 | 40 | 5 |
| Comp. Ex. 2 | glycine | 5 | 40 | 11 |

As shown in Table 1 and Table 2, hemolysis occurred in the blood retention tool containing the 5% glycine (Comparative Example 2). In contrast, in the blood retention tools according to Example 2 (2-1 to 2-5), the hemolysis was prevented from occurring and besides, the development distances of the blood cells were smaller than that in the blood retention tool according to Comparative Example 2, while the amounts of the hemolysis inhibitors contained in the respective blood retention tools were the same as or smaller than that in the blood retention tool according to Comparative Example 2. These results demonstrate that, according to the blood retention tools of the present example containing the above hemolysis inhibitors, blood cells and plasma can be separated sufficiently due to the suppression of the development of the blood cells and the promotion of the development of the plasma. This increases the developing amount of the plasma, thereby improving the collection rate of the plasma.

### (Example 3 and Comparative Example 3)

Blood retention tools (Example 3-1 and 3-2) were produced in the same manner as in Example 1, except that the following development porous membranes (a filter paper and a cellulose acetate membrane) and blood cell separation porous membranes (glass filters) were used and valine was used as the hemolysis inhibitors. Furthermore, blood retention tools according to Comparative Example 3 (Comparative Examples 3-1 and 3-2) were produced in the same manner as in Example 3-1 or 3-2, except that no valine was contained in the blood cell separation porous membranes. Using these blood retention tools, blood cell separation was carried out and the presence or absence of hemolysis and the development of blood cells were determined in the same manner as in Examples 1 and 2. The results are shown in Table 3 below.

| (Filter paper) | |
|---|---|
| Product name: | 3 MMchr (Whatman plc.) |
| Material: | cellulose |
| Thickness: | 340 µm |
| Weight: | 185 g/mm² |
| Porosity: | 20 sec/100 ml/inch² |

| (Cellulose acetate membrane) | |
|---|---|
| Product name: | C300A293C (Toyo Roshi Kaisha, Ltd.) |
| Material: | cellulose acetate |
| Thickness: | 185 µm |
| Weight: | 40 g/mm² |

### (Blood cell separation porous membrane)

A glass filter (product name "AP25": Millipore Corporation) having a thickness of 1200 µm was used.

**(Table 3)**

| | | presence of valine | hemolysis | development distance of blood cells (mm) |
|---|---|---|---|---|
| Ex. 3-1 | filter paper + glass filter | + | none | 8 |
| Comp. Ex. 3-1 | filter paper + glass filter | - | occurred | 14 |
| Ex. 3-2 | cellulose acetate + glass filter | + | none | 3 |
| Comp. Ex. 3-2 | cellulose acetate + glass filter | - | occurred | 6 |

A shown in Table 3, hemolysis occurred in the blood retention tools of Comparative Examples 3-1 and 3-2 in which no valine was contained in the glass filters. In contrast, hemolysis did not occur in the blood retention tools of both Examples 3-1 and 3-2. Moreover, the development distance of the blood cells in the blood retention tool of Example 3-1 was shorter than that in the blood retention tool of Comparative Example 3-1, and the development distance of the blood cells in the blood retention tool of Example 3-2 was shorter than that in the blood retention tool of Comparative Example 3-2. These results demonstrate that, according to the blood retention tools of Example 3, the development of blood cells can be suppressed, so that only the developing amount of plasma can be increased and thus the collection rate of plasma can be improved, as in the case of the blood retention tools of Example 2. It should be noted that the reason why the development distance of the blood cells in the blood retention tool of Examples 3-1 was different from that in the blood retention tool of Example 3-2 is that the types of the development porous membranes used therein were different.

### (Example 4 and Comparative Example 4)

Blood retention tools were produced in the same manner as in Example 1, except that valine solutions with various concentrations (5%, 2%, and 1%) and a 2% leucine solution respectively were used as the hemolysis inhibitors. With regard to each hemolysis inhibitor, ten blood retention tools were produced. Using these blood retention tools, plasma was collected and components contained in the collected plasma were measured (Example 4).

Furthermore, blood retention tools according to Comparative Example 4 were produced in the same manner as in Example 1, except that glycine solutions with predetermined concentrations (20%, 5%, and 0%) were contained in the glass filters, respectively. With regard to each glycine solution, ten blood retention tools were produced.

A whole blood specimen was dropped on each of the blood retention tools to cause the separation of blood cells and the development of plasma. After the plasma had developed, a portion of the development porous membrane where only the plasma had developed was cut out to obtain a cup piece of 10 mm × 6 mm. With regard to each hemolysis inhibitor, ten cut pieces were collected, and they were centrifuged (15,000 g, 10 min) directly to collect the plasma. Using the thus-collected plasma as a sample, the amounts of respective components were measured. Furthermore, as a control test, the amounts of the respective components were measured using the plasma obtained by centrifuging the above whole blood specimen (3000 g, 10 min) as a sample.

### (Measurement of amounts of components)

Amounts of the respective components were measured with an autoanalyzer (product name "Hitachi 7070": Hitachi, Ltd.), using the following commercially available kits according to their application methods. In the respective measurements, purified water was used as a blank.
1. High-Density Lipoprotein Cholesterol (HDL-C)
   Product name: LIQUI TECH HDL-C (Roche Diagnostics K.K.)
2. Amylase (AMY)
   Product name: AMY-NP (DENKA SEIKEN Co.,Ltd.)
3. Albumin (ALB)
   Product name: Clinimate albumin (Daiichi Pure Chemical Co., Ltd.) 4. Glutamic-Oxaloacetic Transaminase (GOT)
   Product name: LIQUI TECH GOT IFCC (Roche Diagnostics K.K.)
5. Gamma Glutamyl Transpeptidase (GGT)
   Product name: γ-GTP-J-HA Wako (Wako Pure Chemical Industries, Ltd.)
6. Glutamic-Pyruvic Transaminase (GPT)
   Product name: LIQUI TECH GPT IFCC (Roche Diagnostics K.K.)
7. Triglyceride (TG)
   Product name: LIQUI TECH TG (Roche Diagnostics K.K.)
8. Lactate Dehydrogenase (LDH)
   Product name: LDH II-HA Wako (Wako Pure Chemical Industries, Ltd.)
9. Total Protein (TP)
   Product name: RD Total Protein (Roche Diagnostics K.K.)
10. Total Bilirubin (T-Bil
   Product name: T-Bil-V5 (AZWELL Inc.)
11. Creatinine (CRE)
   Product name: LIQUI TECH Creatinine PAP (Roche Diagnostics K.K.)
12. Total Cholesterol (TC)
   Product name: T-Cho-KL (International Regents Corporation)
13. Alkaline Phosphatase (ALP)
   Product name: Alkaline Phospha II-HR Test Wako (Wako Pure Chemical Industries, Ltd.)
14. Urea Nitrogen (BUN)
   Product name: Urea Nitrogen II-HA Test Wako (Wako Pure Chemical Industries, Ltd.)
15. Uric Acid (UA)
   Product name: Uricolor - liquid uric acid (Ono Pharmaceutical Co., Ltd.)
16. Fructosamine (FRA)
   Product name: LIQUI TECH Fructosamine (Roche Diagnostics K.K.)
17. Glucose (Glu)
   Product name: LIQUI TECH Glucose (Roche Diagnostics K.K.)
18. Creatinine Phosphokinase (CPK)
   Product name: CK E-HA test Wako (Wako Pure Chemical Industries, Ltd.)

Table 4 below shows the results of the measurements, in which the measured values obtained in Example 4 and Comparative Example 4 are indicated as relative values (percentages on average) with respect to the measured values obtained in the control test as 100%.

**(Table 4)**

| | * | HDL | AMY | ALB | GOT | GGT | GPT | TG | LDH | TP | T-Bil | CRE | TC | ALP | BUN | UA | FRA | Glu | CPK | Average |
|---|---|---|---|---|---|---|---|---|---|---|---|---|---|---|---|---|---|---|---|---|
| ** | 0 | 111% | 106% | 113% | 133% | 108% | 110% | 121% | 146% | 109% | 129% | 109% | 117% | 112% | 113% | 105% | 128% | 90% | 122% | 116% |
| Gly | 2 | 73% | 65% | 75% | 89% | 42% | 90% | 77% | 96% | 70% | 43% | 65% | 67% | 66% | 97% | 70% | 83% | 84% | 83% | 74% |
| | 5 | 90% | 106% | 94% | 120% | 68% | 100% | 96%. | 121% | 86% | 86% | 82% | 87% | 83% | 110% | 85% | 108% | 91% | 96% | 94% |
| Val | 5 | 96% | 100% | 100% | 101% | 93% | 100% | 106% | 103% | 97% | 90% | 92% | 97% | 98% | 103% | 95% | 105% | 97% | 96% | 98% |
| | 2 | 98% | 100% | 102% | 100% | 92% | 96% | 106% | 104% | 94% | 90% | 109% | 100% | 102% | 106% | 96% | 105% | 94% | 103% | 100% |
| | 1 | 103% | 106% | 106% | 111% | 92% | 100% | 113% | 116% | 101% | 86% | 109% | 105% | 108% | 110% | 95% | 118% | 97% | 110% | 105% |
| Leu. | 2 | 107% | 100% | 113% | 112% | 92% | 100% | 121% | 110% | 109% | 86% | 82% | 112% | 109% | 122% | 100% | 123% | 93% | 117% | 106% |

| | | | | | | | | | | | | | | | | | | | | |
|---|---|---|---|---|---|---|---|---|---|---|---|---|---|---|---|---|---|---|---|---|
| * = Concentration (%) ** = Unimpregnated | | | | | | | | | | | | | | | | | | | | |

As shown in Table 4, according to the blood retention tools of Comparative Example 4 containing glycine, variations were observed in the measurement accuracy of the respective components. In particular, with regard to GGT, the relative value with respect to the control test was only about 50%. In contrast, according to the blood retention tools containing valine or leucine, excellent measurement results were obtained with regard to the respective components including GGT. Moreover, hemolysis was prevented in all the blood retention tools of Example 4, whereas the occurrence of hemolysis was observed in the blood retention tools of Comparative Example 4 containing the 5% glycine solution. Furthermore, the blood retention tools of Comparative Example 4 containing the 20% glycine solution exhibited small relative values with regard to all the components. The reason for this is considered to be that blood cells shrank due to the high concentration of glycine, so that the volume of the plasma components increased accordingly, thereby diluting the plasma components. These results demonstrate that the blood retention tools according to the present invention can prevent hemolysis and also can prepare a sample causing little influence on the measurement of the respective components.

### Industrial Applicability

As described above, according to the blood cell separation membrane of the present invention, it is possible to separate blood into blood cells and serum/plasma while preventing the occurrence of hemolysis of the blood cells, so that a serum or plasma sample can be prepared efficiently Moreover, a sample prepared by the blood cell separation membrane of the present invention has little influence on measurement systems of various components. Thus, highly accurate measurement becomes possible by using such a sample. Therefore, the blood cell separation membrane and the blood retention tool of the present invention are useful in fields such as clinical medicine as described above, for example.

## Claims

1. A blood cell separation membrane comprising a porous membrane for separating blood into blood cells and serum or plasma,
wherein the porous membrane contains at least one hemolysis inhibitor selected from the group consisting of valine, leucine, silk extracts, ε-aminohexanoic acid, and tranexamic acid,
wherein a content of the hemolysis inhibitor is in a range from 1 mg to 50 mg per unit volume (cm³) of the porous membrane.

2. The blood cell separation membrane according to claim 1, wherein the porous membrane is at least one of a glass filter and an asymmetric porous membrane with a pore size distribution in which an average pore size varies so as to be reduced continuously or discontinuously in a thickness direction.

3. The blood cell separation membrane according to claim 2, wherein a material of the asymmetric porous membrane is at least one resin selected from the group consisting of polysulfone, polyethersulfone, polyamide, polyimide, polycarbonate, polystyrene, and polyaryl hydrazide.

4. The blood cell separation membrane according to claim 2 or 3, wherein in the asymmetric porous membrane, a maximum pore size is in a range from 30 to 300 µm and a minimum pore size is in a range from 1 to 30 µm.

5. The blood cell separation membrane according to any one of claims 1 to 6, wherein the hemolysis inhibitor is contained on at least one surface side of the blood cell separation membrane.

6. A blood retention tool comprising:
a blood cell separation portion for separating blood cells from blood; and
a development portion in which serum or plasma contained in the blood develops and is collected or treated,
wherein the blood cell separation portion is the blood cell separation membrane according to any one of claims 1 to 5

7. The blood retention tool according to claim 6, wherein the blood cell separation portion is laminated on the development portion.

8. The blood retention tool according to claim 8 or 7, wherein the development portion is a porous membrane.

9. The blood retention tool according to claim 6, wherein the blood cell separation membrane includes a blood cell separation portion and a development portion.

10. The blood retention tool according to claim 8, wherein a material of the porous membrane is at least one resin selected from the group consisting of polysulfone, polyethersulfone, polyamide, polyimide, polycarbonate, polystyrene, and polyaryl hydrazide.

11. The blood retention tool according to claim 6, wherein the blood cell separation portion is an asymmetric porous membrane,
the development portion is provided in the asymmetric porous membrane, and
the blood cell separation portion and the development portion are arranged along a surface direction of the asymmetric porous membrane so that the blood cell separation portion is on a side from which blood is supplied and the development portion is on a downstream side in a developing direction of the blood supplied.

12. The blood retention tool according to claim 11, wherein the blood cell separation portion is the asymmetric porous membrane,
the development portion is provided in the asymmetric porous membrane,
the blood cell separation portion and the development portion are arranged along the surface direction of the asymmetric porous membrane, and
a blood cell blocking portion including only pores through which blood cells cannot pass is formed between the blood cell separation portion and the development portion so as to extend in a width direction of the asymmetric porous membrane.

13. The blood retention tool according to claim 12, wherein a groove is formed so as to extend in the width direction of the asymmetric porous membrane, and a portion between a bottom of the groove and a part of a surface of the asymmetric porous membrane corresponding to the bottom serves as the blood cell blocking portion.

14. The blood retention tool according to any one of claims a to 13. wherein a reagent portion containing an analytical reagent is formed in the development portion.

15. The blood retention tool according to any one of claims 6 to 14, further comprising a reagent layer containing a an analytical reagent.

## Patentansprüche

1. Blutzellen-Separationsmembran, mit einer porösen Membran, um Blut in Blutzellen und Serum oder Plasma zu separieren,
wobei die poröse Membran wenigstens einen Hämolyse-Inhibitor enthält, der aus der Gruppe gewählt ist, die aus Valin, Leucin, Seidenextrakten, ε-Cabronsäure und Tranexamic-Säure besteht,
wobei ein Gehalt eines Hämolyse-Inhibitors in einem Bereich von 1 mg bis 50 mg pro Einheitsvolumen (cm³) der porösen Membran liegt.

2. Blutzellen-Separationsmembran nach Anspruch 1, wobei die poröse Membran ein Glasfilter und/oder eine asymmetrische poröse Membran mit einer Porengrößenverteilung, bei der eine durchschnittliche Porengröße so variiert, dass sie in einer Dickenrichtung kontinuierlich oder diskontinuierlich abnimmt, ist.

3. Blutzellen-Separationsmembran nach Anspruch 2, wobei ein Material der asymmetrischen porösen Membran wenigstens ein Harz ist, das aus der Gruppe gewählt ist, die aus Polysulfon, Polyethersulfon, Polyamid, Polyimid, Polycarbonat, Polystyrol und Polyaryl-Hydracid besteht.

4. Blutzellen-Separationsmembran nach Anspruch 2 oder 3, wobei in der asymmetrischen porösen Membran eine maximale Porengröße in einem Bereich von 30 bis 300 µm liegt und eine minimale Porengröße in einem Bereich von 1 bis 30 µm liegt.

5. Blutzellen-Separationsmembran nach einem der Ansprüche 1 bis 6, wobei der Hämolyse-Inhibitor wenigstens auf einer Oberflächenseite der Blutzellen-Separationsmembran enthalten ist.

6. Blutrückhaltewerkzeug mit:
einem Blutzellen-Separationsabschnitt, um Blutzellen von Blut zu separieren; und
einem Entwicklungsabschnitt, in dem sich Serum oder Plasma, das in dem Blut enthalten ist, entwickelt und gesammelt oder behandelt wird,
wobei der Blutzellen-Separationsabschnitt die Blutzellen-Separationsmembran nach einem der Ansprüche 1 bis 5 ist.

7. Blutrückhaltewerkzeug nach Anspruch 6, wobei der Blutzellen-Separationsabschnitt auf den Entwicklungsabschnitt laminiert ist.

8. Blutrückhaltewerkzeug nach Anspruch 6 oder 7, wobei der Entwicklungsabschnitt eine poröse Membran ist.

9. Blutrückhaltewerkzeug nach Anspruch 6, wobei die Blutzellen-Separationsmembran einen Blutzellen-Separationsabschnitt und einen Entwicklungsabschnitt aufweist.

10. Blutrückhaltewerkzeug nach Anspruch 8, wobei ein Material der porösen Membran wenigstens ein Harz ist, das aus der Gruppe gewählt ist, die aus Polysulfon, Polyethersulfon, Polyamid, Polyimid, Polycarbonat, Polystyrol und Polyaryl-Hydracid besteht.

11. Blutrückhaltewerkzeug nach Anspruch 6, wobei der Blutzellen-Separationsabschnitt eine asymmetrische poröse Membran ist,
der Entwicklungsabschnitt in der asymmetrischen porösen Membran vorgesehen ist und
der Blutzellen-Separationsabschnitt und der Entwicklungsabschnitt längs einer Oberflächenrichtung der asymmetrischen porösen Membran so angeordnet sind, dass der Blutzellen-Separationsabschnitt auf einer Seite vorhanden ist, von der Blut zugeführt wird, und der Entwicklungsabschnitt stromabseitig in einer Entwicklungsrichtung des zugeführten Bluts vorhanden ist.

12. Blutrückhaltewerkzeug nach Anspruch 11, wobei der Blutzellen-Separationsabschnitt die asymmetrische poröse Membran ist,
der Entwicklungsabschnitt in der asymmetrischen porösen Membran vorgesehen ist,
der Blutzellen-Separationsabschnitt und der Entwicklungsabschnitt längs der Oberflächenrichtung der asymmetrischen porösen Membran angeordnet sind und
ein Blutzellen-Blockierabschnitt, der nur Poren aufweist, durch die sich Blutzellen nicht bewegen können, zwischen dem Blutzellen-Separationsabschnitt und dem Entwicklungsabschnitt ausgebildet ist, um sich in einer Breitenrichtung der asymmetrischen porösen Membran zu erstrecken.

13. Blutrückhaltewerkzeug nach Anspruch 12, wobei eine Nut so ausgebildet ist, dass sie sich in der Breitenrichtung der asymmetrischen porösen Membran erstreckt, und ein Abschnitt zwischen einem Boden der Nut und einem Teil einer Oberfläche der asymmetrischen porösen Membran, der dem Boden entspricht, als der Blutzellen-Blockierabschnitt dient.

14. Blutrückhaltewerkzeug nach einem der Ansprüche 6 bis 13, wobei ein Reagenzabschnitt, der ein analytisches Reagenz enthält, in dem Entwicklungsabschnitt ausgebildet ist.

15. Blutrückhaltewerkzeug nach einem der Ansprüche 6 bis 14, der ferner eine Reagenzschicht enthält, die ein analytisches Reagenz enthält.

## Revendications

1. Membrane de séparation de cellules sanguines comprenant une membrane poreuse pour séparer le sang en cellules sanguines et sérum ou plasma,
dans laquelle la membrane poreuse contient au moins un inhibiteur d'hémolyse choisi dans le groupe constitué de valine, de leucine, d'extraits de soie, d'acide ε-aminohexanoïque et d'acide tranexamique,
dans laquelle la teneur en inhibiteur d'hémolyse est dans une plage allant de 1 mg à 50 mg par volume unitaire (cm³) de la membrane poreuse.

2. Membrane de séparation de cellules sanguines selon la revendication 1, dans laquelle la membrane poreuse est au moins l'un d'un filtre en verre et d'une membrane poreuse asymétrique avec une distribution de tailles de particules dans laquelle la taille moyenne des pores varie de sorte à être réduite de façon continue ou discontinue dans le sens de l'épaisseur.

3. Membrane de séparation de cellules sanguines selon la revendication 2, dans laquelle une matière de la membrane poreuse asymétrique est au moins une résine choisie dans le groupe constitué de polysulfone, de polyéthersulfone, de polyamide, de polyimide, de polycarbonate, de polystyrène et de polyaryl-hydrazide.

4. Membrane de séparation de cellules sanguines selon la revendication 2 ou 3, dans laquelle à l'intérieur de la membrane poreuse asymétrique, une taille maximum de pores est dans une plage allant de 30 à 300 µm et une taille minimum de pores est dans une plage allant de 1 à 30 µm.

5. Membrane de séparation de cellules sanguines selon l'une quelconque des revendications 1 à 6, dans laquelle au moins un côté de la surface de la membrane de séparation des cellules sanguines contient l'inhibiteur d'hémolyse.

6. Dispositif de rétention de sang comprenant :
une portion de séparation de cellules sanguines pour séparer des cellules sanguines du sang ; et
une portion de développement dans laquelle le sérum ou le plasma contenu dans le sang se développe et est recueilli ou traité,
dans lequel la portion de séparation de cellules sanguines est la membrane de séparation des cellules sanguines selon l'une quelconque des revendications 1 à 5.

7. Dispositif de rétention de sang selon la revendication 6, dans lequel la portion de séparation de cellules sanguines est stratifiée sur la portion de développement.

8. Dispositif de rétention de sang selon la revendication 6 ou 7, dans lequel la portion de production est une membrane poreuse.

9. Dispositif de rétention de sang selon la revendication 6, dans lequel la membrane de séparation de cellules sanguines inclue une portion de séparation de cellules sanguines et une portion de développement.

10. Dispositif de rétention de sang selon la revendication 8, dans lequel une matière de la membrane poreuse est au moins une résine choisie dans le groupe constitué de polysulfone, de polyéthersulfone, de polyamide, de polyimide, de polycarbonate, de polystyrène et de polyaryl-hydrazide.

11. Dispositif de rétention de sang selon la revendication 6, dans lequel la portion de séparation de cellules sanguines est une membrane poreuse asymétrique,
la portion de développement est fournie dans la membrane poreuse asymétrique,
la portion de séparation de cellules sanguines et la portion de développement sont agencées le long d'une direction de surface de la membrane poreuse asymétrique de sorte que la portion de séparation de cellules sanguines est du côté à partir duquel le sang est mis à disposition et la portion de développement est sur un côté aval en une direction de développement du sang mis à disposition.

12. Dispositif de rétention de sang selon la revendication 11, dans lequel la portion de séparation de cellules sanguines est la membrane poreuse asymétrique,
la portion de développement est fournie dans la membrane poreuse asymétrique,
la portion de séparation de cellules sanguines et la portion de développement sont agencées le long de la direction de la surface de la membrane poreuse asymétrique, et
une portion de blocage de cellules sanguines incluant seulement des pores au travers desquels les cellules sanguines ne peuvent pas passer se forme entre la portion de séparation de cellules sanguines et la portion de développement de façon à s'étendre dans une direction de largeur de la membrane poreuse asymétrique.

13. Dispositif de rétention de sang selon la revendication 12, dans lequel un sillon est formé de façon à s'étendre dans une direction de la largeur de la membrane poreuse asymétrique, et une portion entre le fond du sillon et une partie de la surface de la membrane poreuse asymétrique correspondant au fond sert de portion de blocage des cellules sanguines.

14. Dispositif de rétention de sang selon l'une quelconque des revendications 6 à 13, dans lequel une portion de réactif contenant un réactif analytique est formé dans la portion de développement.

15. Dispositif de rétention de sang selon l'une quelconque des revendications 6 à 14, comprenant en outre une couche de réactif contenant un réactif analytique.
